# EUROPEAN PATENT APPLICATION

(11) **EP 4 282 337 A1**
(43) Date of publication of application: **29.11.2023**
(21) Application number: 22175698.4
(22) Date of filing: 27.05.2022
(51) Int. Cl.: A61B 6/03, A61B 6/00, A61G 3/00

(54) **MEDICAL VEHICLE AND METHOD FOR OPERATING A MEDICAL VEHICLE**

(71) Applicant: Koninklijke Philips N.V., 5656 AG Eindhoven (NL)
(72) Inventor: JOHNSON, Mark Thomas, Eindhoven (NL); BUSSA, Nagaraju, Eindhoven (NL); VOGTMEIER, Gereon, 5656AG Eindhoven (NL); MYSORE SIDDU, Dinesh, 5656AG Eindhoven (NL)
(74) Representative: Philips Intellectual Property & Standards

(57) **Abstract**

The invention relates to a medical vehicle (1) comprising a base vehicle (2) and a compartment (3) with a medical imaging system (4). The compartment (3) is a part of the base vehicle (2) or the compartment (3) is attached to the base vehicle (2). The base vehicle (2) comprises at least one base vehicle sensor (5) and the medical vehicle (1) is configured to obtain sensor readings from the at least one base vehicle sensor (5) during operation of the medical vehicle (1) and perform medical scans of a patient during operation of the medical vehicle (1) in dependence on the obtained sensor readings. The invention further relates to a corresponding method for operating a medical vehicle (1).

## Description

### FIELD OF THE INVENTION

The invention relates to a medical vehicle comprising a base vehicle and a compartment with a medical imaging system, the medical vehicle being configured to perform medical scans during operation of the medical vehicle. The invention further relates to a method for operating a medical vehicle comprising performing medical scans during operation of the medical vehicle.

### BACKGROUND OF THE INVENTION

Medical vehicles that comprise medical imaging systems may be used for medical scans during transportation. In some cases, like a mobile clinic in a ship, performing medical scans during transportation is the common usage of the medical imaging system. Also, an ambulance truck, equipped with a medical imaging system, may already perform medical scans of a patient in order to save precious time, e.g., a plane CT scan may be performed for a stroke assessment or scans of the heart may be performed for the assessment of a heart attack.

However, during transportation, the medical imaging system is subjected to adverse conditions such as impact forces caused by the motion of the medical vehicle which may cause errors in scanning. Said errors may be caused by a motion of the patient due to the impact forces, which may require a re-calibration of the medical imaging system, and/or by damage to and/or motion of the medical imaging system.

### SUMMARY OF THE INVENTION

It is therefore an object of the present invention to provide a medical vehicle with an improved ability to perform medical scans during operation of the medical vehicle. It is a further object of the present invention to provide an improved method for operating a medical vehicle that comprises performing medical scans during operation of the medical vehicle.

The object of the present invention is solved by the subject matter of the independent claims, wherein further embodiments are incorporated in the dependent claims.

In an aspect of the present invention, a medical vehicle comprising a base vehicle and a compartment with a medical imaging system is provided. The main purpose of the medical vehicle may be the medical imaging, i.e., performing medical scans using the medical imaging system. Here, the medical vehicle may be optimized to perform medical scans during operation of the medical vehicle, and/or it may be optimized to perform medical scans when the medical vehicle is stopped while being configured to perform medical scans during operation of the medical vehicle. Alternatively, or additionally, the main purpose of the medical vehicle may be a non-medical one, such as a cruise ship that has a medical imaging system as part of its medical equipment.

The base vehicle is the vehicle transporting the compartment with the medical imaging system, i.e., the base vehicle is the medical vehicle without the compartment with the medical imaging system. As above, the base vehicle may be specifically configured for the use with the medical imaging system, such as a truck with a medical imaging system, and/or the base vehicle may have a non-medical main purpose, such as a cruise ship.

The compartment is a part of the base vehicle or is attached to the base vehicle. In other words, the compartment may be a virtual compartment defined by a volume of an existing compartment of the base vehicle or may comprise physical walls. In the latter case, the compartment may be permanently attached to the base vehicle or may be detachable such that it can be placed separately from the base vehicle. The compartment may be just large enough to fit the medical imaging system, but it may also provide extra space next to the medical imaging system, e.g., for medical staff.

The base vehicle comprises at least one base vehicle sensor. Said base vehicle sensor may be used to assess adverse conditions for the medical imaging system.

The medical vehicle is configured to obtain sensor readings from the at least one base vehicle sensor during operation of the medical vehicle. In this context, operation may refer to travel of the medical vehicle but may also encompass the medical vehicle being temporarily stationary during a general travel of the vehicle, e.g., when the vehicle is stopped at a traffic light, at a railroad crossing or in a traffic jam. If the base vehicle sensor is already present in the base vehicle, there is no need to add an extra sensor, which may result in considerable cost savings.

The medical vehicle is further configured to perform medical scans of a patient during operation of the medical vehicle in dependence on the obtained sensor readings. In this context, the patient may be a person or an animal. By performing the medical scans during operation of the medical vehicle, precious time can be saved for the patient, e.g., after a stroke or after a heart attack. In the case of a ship, performing the medical scans during operation of the medical vehicle is the only reasonable option of performing a medical scan. By performing the medical scans in dependence on the obtained sensor readings, the quality and/or significance of the medical scans is improved, hence leading to a better and/or faster diagnosis, which, in turn, leads to a better and/or faster treatment of the patient.

According to an embodiment, the base vehicle is a truck, a train, a plane, a helicopter, an autonomous flight object and/or a ship. In all of said vehicles, a medical imaging system may be installed, transported and used during operation of the medical vehicle. Adverse conditions may stem from, e.g., potholes, corners, hills, turbulences, waves or environmental conditions and may be assessed using the sensor readings from the at least one base vehicle sensor.

According to an embodiment, the medical imaging system is a magnetic resonance imaging (MRI), a computed tomography (CT), a digital X-ray radiogrammetry (DXR), a single-photon emission computed tomography (SPECT), a positron emission tomography (PET), a dark field X-ray imaging, a small dedicated medical imaging and/or an ultrasound system. Each of these systems is very sensitive to adverse conditions such as impact forces acting upon it and hence it is very valuable to improve performing medical scans during operation of the medical vehicle in dependence on the obtained sensor readings.

According to an embodiment, the at least one base vehicle sensor is a vehicle navigation system, an autonomous driving system, a board computer, a compass, a camera, a radar, a lidar, a speedometer, a wind sensor, a wave sensor, an acceleration sensor, an inertial measurement unit (IMU), a motion control sensor, an engine control sensor, a vibration sensor, a temperature sensor, a humidity sensor and/or an electromagnetic field sensor. As an example, the vehicle navigation system may provide information on road conditions ahead, such as curves, inclinations or even road bumps, wherein said information may, for example, also be used for driver assistance systems and/or for autonomous driving. Further, the vehicle navigation system may provide information on traffic conditions ahead, e.g., on traffic jams. The autonomous driving system can further provide information on planned or predicted actuator data, e.g., on accelerations, on decelerations and on curves. The camera, radar and/or lidar may also detect road conditions ahead for a land vehicle or water conditions for a sea vehicle. The engine control sensor may provide, e.g., information on the r.p.m. of the engine, which may be used to predict resonant vibrations. The vibration sensor may detect vibrations of the base vehicle and/or of the compartment. Further, the temperature sensor, the humidity sensor and/or the electromagnetic field sensor may measure the temperature, humidity and/or electromagnetic fields, respectively, outside of the vehicle and/or inside the vehicle, in particular inside the compartment.

According to an embodiment, performing medical scans during operation of the medical vehicle in dependence on the sensor readings comprises predicting adverse conditions on the compartment based on the obtained sensor readings and scheduling medical scans based on the predicted adverse conditions.

In this context, the adverse conditions for the medical imaging system may be impact forces on the compartment and adverse environmental conditions in the compartment. Impact force may be any force other than the gravitational force acting in a predetermined downwards direction. In particular, impact forces may be forces due to an acceleration of the compartment housing the medical imaging system. As examples, impact forces may be forces caused by bumps in the road, may be centrifugal forces when the medical vehicle changes direction or may be caused by tilting the compartment, such that the direction of the gravitational force changes. The environmental conditions may be, e.g., temperature, humidity, atmospheric pressure and/or electromagnetic fields and become adverse environmental conditions when said parameters are outside a predetermined range.

Said adverse conditions are predicted based on the obtained sensor readings. For example, the vehicle navigation system may provide information on the road conditions ahead, including curves and/or even bumps. In combination with the vehicle speed obtained from either the vehicle navigation system or the speedometer, impact forces on the medical vehicle and on the compartment may be predicted. In an autonomous driving mode, the autonomous driving system may further provide information on planned actuator data, i.e., planned accelerations, decelerations and/or curves, that can be used to further improve the prediction of impact forces. Additionally or alternatively, the road conditions ahead may also be assessed by a camera, a radar and/or a lidar, leading to a prediction of impact forces on the medical vehicle and on the compartment. As another example, a temperature map may be provided by the vehicle navigation system, hence giving a prediction of outside temperatures which may also affect the temperatures inside the compartment.

The medical scans are then performed based on the predicted adverse conditions such that the highest quality and/or significance of the medical scans may be achieved.

According to an embodiment, performing medical scans during operation of the medical vehicle in dependence on the sensor readings comprises determining, based on the obtained sensor readings, current adverse conditions on the compartment and performing and/or dynamically adapting medical scans based on the determined current adverse conditions.

Determining current adverse conditions may be performed, e.g., by evaluating readings from the acceleration sensor or the inertial measurement unit for impact forces or by evaluating readings from the temperature sensor, the humidity sensor and/or the electromagnetic field sensor for adverse environmental conditions. Determining the current adverse conditions may also be performed by analyzing information provided by the board computer: for example, turned on hazard warning lights and/or an engaged parking brake may indicate that the medical vehicle is at rest and hence the impact forces are negligible. Also, an engaged cruise control, speed limiter, lane guidance assistant and/or r.p.m. control may indicate that the vehicle is travelling rather smoothly and hence the impact forces are below a predetermined value. Further, the board computer may indicate that an accelerator or brakes are used, that a steering wheel is turned and/or that a driver assistance system has been disabled, indicating that there will be substantial impact force, exceeding a predetermined value.

The medical scans are then performed based on the determined current adverse conditions such that the highest quality and/or significance of the medical scans may be achieved. Additionally, or alternatively, the medical scans are adapted dynamically based on the determined current adverse conditions. As an example, said adaptation may be a variation in exposure time, such that the greater the impact forces, the shorter the exposure times to still produce a sharp medical image. As another example, the adaptation may be a change in resolution, such that greater the impact forces, the coarser the resolution of the medical image.

According to an embodiment, the scheduling and/or performing of the medical scans is done such that the medical scans that are most sensitive to adverse conditions are performed when the least adverse conditions are predicted and/or determined. For this, the sensitivity of the medical scans on the adverse conditions has been determined beforehand. In particular, there may be scans with different resolutions that have different sensitivity on the adverse conditions and/or even the scans from one scan sequence may have different sensitivity on the adverse conditions. As an example, when it is determined and/or predicted that the medical vehicle is or will be stopped, e.g., by information provided that a parking brake is engaged or by information provided by the navigation system that a traffic jam is ahead, the medical scan having the most sensitivity to impact forces will be performed at and/or scheduled for that time. As another example, when moderate impact forces are determined and/or predicted, a scan having lesser sensitivity to impact forces will be performed at and/or scheduled for that time. And when substantial impact forces are determined and/or predicted, the medical scans may be suspended for the duration of said substantial impact forces. Hence, an optimal match between the medical scans and the adverse conditions is found and an optimal quality of the medical scans is achieved.

According to an embodiment, the scheduling and/or performing of the medical scans is done such that that medical scan out of remaining medical scans, in particular of a scan sequence, is performed that has, among those medical scans that are allowed by the predicted and/or determined adverse conditions, the highest sensitivity to adverse conditions. In other words, based on the determined and/or predicted adverse conditions, it is determined which medical scans of the remaining scans are allowed to be performed, and among those medical scans, the one is chosen that has the highest sensitivity to the adverse conditions. In this context, "allowed" to be performed may mean that the medical scan may be performed without loss of quality compared to the same medical scan at ideal conditions, i.e., without adverse conditions. Hence, all of the medical scans that have been performed have the highest possible quality, but some of the medical scans of the scan sequence may not have been performed at all. These scans may, however, then be performed at, for example, a hospital, such that the scan sequence is completed.

According to an embodiment, the scheduling and/or performing of the medical scans is done such that the adverse conditions are most comparable to adverse conditions of a previous scan. In particular, the new medical scan is then an update scan or a comparison scan, to assess possible changes that may have occurred since the previous scan. When the most comparable adverse conditions are chosen for the update scan, the impact of the adverse conditions on the medical scans are similar such that the update scan can most easily be compared to the previous scan.

According to an embodiment, performing medical scans during operation of the medical vehicle in dependence on the obtained sensor readings comprises adapting settings of the medical vehicle in dependence on the obtained sensor readings. As an example, vibration compensators may be activated when certain vibrations are detected or when the r.p.m. of the engine are in a range known to cause resonant vibrations. As another example, the vehicle speed may be limited such that road bumps or curves lead to smaller impact forces. As yet another example, a vehicle route may be changed such that the vehicle travels on a smoother and/or less curvy road. As yet another example, settings of a vehicle suspension system may be adjusted in order to lessen the impact forces caused by road bumps. As yet another example, settings of an anchoring system that anchors the medical imaging system to the compartment, may be adjusted. Said anchoring system may be, for example, in a locking mode where the medical imaging system is locked to the compartment, in a suspension mode where there is a suspension such as a spring suspension that allows limited motion of the medical imaging system and absorbs some of the impact forces that act on the compartment, or in a damping mode, where said suspension is complemented by a damping system such as shock absorbers to prevent oscillations. If both the vehicle suspension system and the anchoring system are adjusted, a control unit may be used to coordinate the adjustments of these systems, in particular in order to avoid overcompensation and to avoid resonant oscillations of the medical imaging system. As yet another example, environmental conditioning settings, e.g., air conditioning settings, of the vehicle may be adjusted when temperatures and/or humidities have been determined to be out of a predetermined range.

According to an embodiment, the medical vehicle is further configured to prevent the change of vehicle settings while an extra sensitive scan is performed. In this context, an extra sensitive scan is a medical scan that has been identified to be very susceptible to adverse conditions. For such an extra sensitive scan, a change of vehicle settings, such as a hardening of the suspension, while the scan is performed would most likely lead to unusable scan results. In particular, if no impact forces are allowed during such an extra sensitive scan, the extra sensitive scan will be started only when the vehicle is at rest, and starting to drive again would lead to unusable scan results. Hence, in that case, a change of the vehicle from rest to a driving state would be prevented. Said prevention of the change of vehicle settings may be performed, for example, by sending a first message from the medical imaging system to a vehicle control unit indicating that the extra sensitive scan has started and that the vehicle settings must not be changed and sending another message from the medical imaging system to the vehicle control unit indicating that the extra sensitive scan has been completed and that the vehicle settings may be changed again.

According to an embodiment, performing medical scans during operation of the medical vehicle in dependence on the obtained sensor readings comprises determining a movement of the compartment, the medical imaging system, a part of the medical imaging system and/or the patient based on the obtained sensor readings. In particular, said determination of a movement may be performed by an accelerometer and/or an inertial measurement unit. The determined acceleration can then be used to compute the movement of the compartment and based on the movement of the compartment, a movement of the medical imaging system, of parts of the medical imaging system and/or of the patient may be inferred. A movement of the patient with respect to the medical imaging system would naturally affect the medical scans, but also a movement of parts of the medical imaging system may lead to deformations of the medical imaging system and hence, e.g., to a shift of a focus point.

According to an embodiment, the medical vehicle is further configured to dynamically adapt a medical image formation based on the determined movement of the compartment, the medical imaging system, the part of the medical imaging system and/or the patient. As an example, said dynamical adaptation may comprise a compensation calculation based on the determined movement. In this example, the movement may be, e.g., a movement of the patient, a vibration of the medical imaging system that leads to a reduced spatial resolution and/or a bending of parts of the medical imaging system, resulting, e.g., in a shift of the focus point. As another example, said dynamical adaptation may comprise an active shift of the patient to counteract the determined movement.

According to an embodiment, the medical vehicle is further configured to store the determined movement of the compartment, the medical imaging system, the part of the medical imaging system and/or the patient for use for compensation of the movement in post processing of the medical images. Hence, the determined movements can be retrospectively be compensated for. As above, the determined movements may be, e.g., a movement of the patient, a vibration of the medical imaging system that leads to a reduced spatial resolution and/or a bending of parts of the medical imaging system, resulting, e.g., in a shift of the focus point.

If more than one of the above embodiments are realized in the medical vehicle, a selection may have to be made which one or which ones of them is/are applied in a case at hand. Such selection may be made manually, i.e., by an operator of the medical vehicle or of the medical imaging system, using a predefined priority sequence and/or using an algorithm, in particular an algorithm that is optimized by artificial intelligence. Said selection methods may further depend on the patient, the medical condition of the patient and/or details of the medical imaging to be performed on the patient.

In another aspect of the present invention, a method for operating a medical vehicle according to the above description is provided. The method comprises obtaining sensor readings from the at least one base vehicle sensor during operation of the medical vehicle and performing medical scans of the patient during operation of the medical vehicle in dependence on the obtained sensor readings. By performing the medical scans in dependence on the obtained sensor readings, the quality and/or significance of the medical scans is improved, hence leading to a better and/or faster diagnosis, which, in turn, leads to a better and/or faster treatment of the patient. Further details, examples and advantages are provided in the above description.

It shall be understood that a preferred embodiment of the invention can also be any combination of the dependent claims with the respective independent claim.

These and other aspects of the invention will be apparent from and elucidated with reference to the embodiments described hereinafter.

### BRIEF DESCRIPTION OF THE DRAWINGS

In the following, preferred embodiments of the invention will be described, by way of example only, and with reference to the drawings in which:
Fig. 1 shows a schematical side view of a medical vehicle;
Fig. 2 shows a schematical side view of another embodiment of a medical vehicle; and
Fig. 3 shows a flowchart of a method for operating a medical vehicle.

### DETAILED DESCRIPTION OF EMBODIMENTS

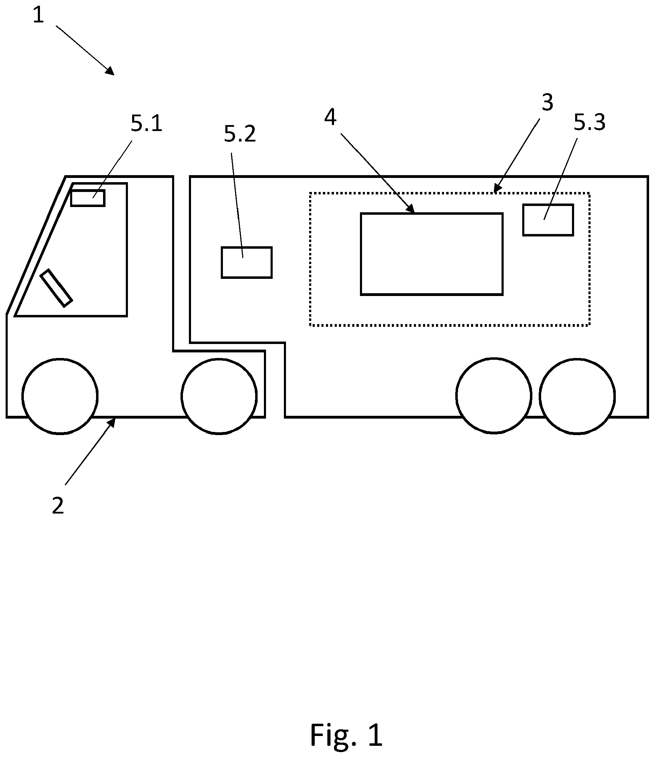

Fig. 1 shows a schematical side view of a medical vehicle 1. The medical vehicle 1 is depicted as a truck, but may as well be a train, a plane, a helicopter, an autonomous flight object or a ship.

The medical vehicle 1 comprises a base vehicle 2 and a compartment 3 with a medical imaging system 4. The compartment 3 may be a part of the base vehicle 2 or may be attached to the base vehicle 2. The medical imaging system 4 may be a magnetic resonance imaging (MRI), a computed tomography (CT), a digital X-ray radiogrammetry (DXR), a single-photon emission computed tomography (SPECT), a positron emission tomography (PET), a dark field X-ray imaging, a small dedicated medical imaging and/or an ultrasound system.

The base vehicle 2 comprises a plurality of base vehicle sensors 5, for example a camera 5.1, an inertial measurement unit 5.2 and a temperature sensor 5.3. Other base vehicle sensors 5 may be a vehicle navigation system, an autonomous driving system, a board computer, a compass, a radar, a lidar, a speedometer, a wind sensor, a wave sensor, an acceleration sensor, a motion control sensor, an engine control sensor, a vibration sensor, a humidity sensor and an electromagnetic field sensor.

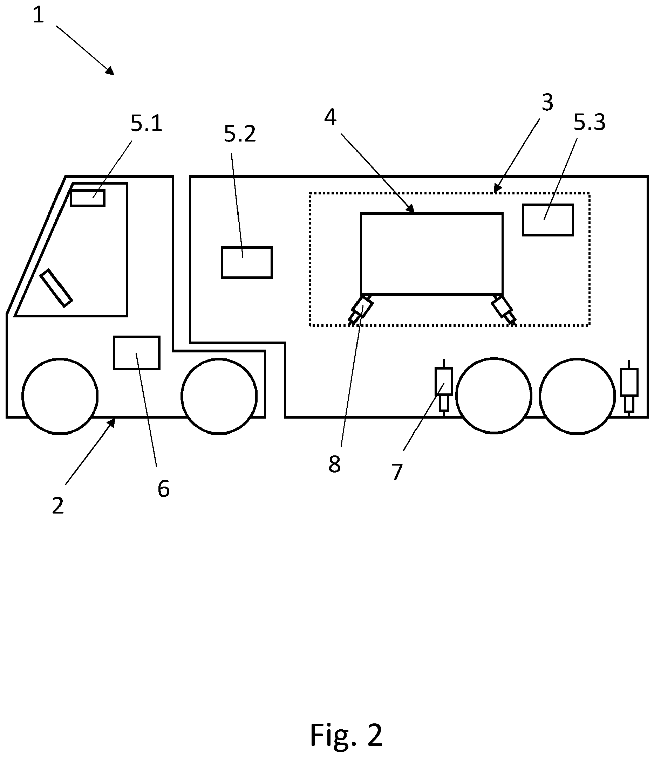

Fig. 2 shows a schematical side view of another embodiment of a medical vehicle 1. In addition to the features of the embodiment of Fig. 1, the base vehicle 2 of Fig. 2 further comprises a vehicle management system 6 that is configured to adapt and/or control vehicle settings. Moreover, the base vehicle 2 comprises an active suspension 7 of the vehicle and an anchoring system 8 which anchors the medical imaging system 4 to the compartment 3.

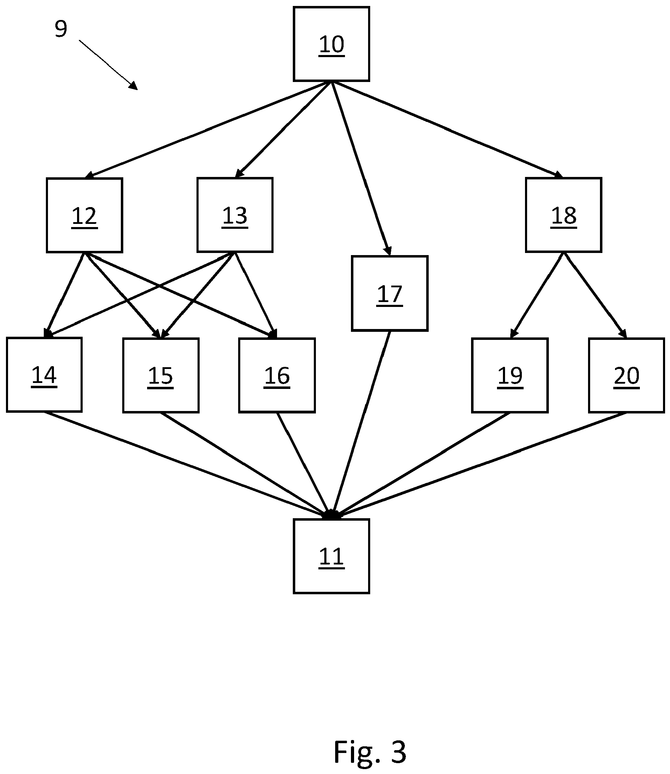

Fig. 3 shows a flowchart of a method 9 for operating a medical vehicle 1. While several options are shown in Fig. 3, all of the options have in common that sensor readings from the at least one base vehicle sensor 5 are obtained 10 and that medical scans of a patient (not shown here) are performed 11 during operation of the medical vehicle 1 in dependence on the obtained sensor readings. Since the medical scans are performed in dependence on the obtained sensor readings, the quality and/or significance of the medical scans is improved, hence leading to a better and/or faster diagnosis, which, in turn, leads to a better and/or faster treatment of the patient.

According to one option, adverse conditions on the compartment 3 are predicted 12 based on the obtained sensor readings. Here, adverse conditions for the medical imaging system 4 may be impact forces on the compartment 3 and adverse environmental conditions in the compartment 3. Impact force may be any force other than the gravitational force acting in a predetermined downwards direction. In particular, impact forces may be forces due to an acceleration of the compartment 3 housing the medical imaging system 4. As examples, impact forces may be forces caused by bumps in the road, may be centrifugal forces when the medical vehicle 1 changes direction or may be caused by tilting the compartment 3, such that the direction of the gravitational force changes. The environmental conditions may be, e.g., temperature, humidity, atmospheric pressure and/or electromagnetic fields and become adverse environmental conditions when said parameters are outside a predetermined range. Said adverse conditions are predicted based on the obtained sensor readings. For example, the vehicle navigation system may provide information on the road conditions ahead, including curves and/or even bumps. In combination with the vehicle speed obtained from either the vehicle navigation system or the speedometer, impact forces on the medical vehicle and on the compartment may be predicted. In an autonomous driving mode, the autonomous driving system may further provide information on planned actuator data, i.e., planned accelerations, decelerations and/or curves, that can be used to further improve the prediction of impact forces. Additionally or alternatively, the road conditions ahead may also be assessed by a camera, a radar and/or a lidar, leading to a prediction of impact forces on the medical vehicle and on the compartment. As another example, a temperature map may be provided by the vehicle navigation system, hence giving a prediction of outside temperatures which may also affect the temperatures inside the compartment.

According to another option, current adverse conditions on the compartment 3 are determined 13, based on the obtained sensor readings. Determining current adverse conditions may be performed, e.g., by evaluating readings from the acceleration sensor or the inertial measurement unit 5.2 for impact forces or by evaluating readings from the temperature sensor 5.3, the humidity sensor and/or the electromagnetic field sensor for adverse environmental conditions. Determining the current adverse conditions may also be performed by analyzing information provided by the board computer: for example, turned on hazard warning lights and/or an engaged parking brake may indicate that the medical vehicle is at rest and hence the impact forces are negligible. Also, an engaged cruise control, speed limiter, lane guidance assistant and/or r.p.m. control may indicate that the vehicle is travelling rather smoothly and hence the impact forces are below a predetermined value. Further, the board computer may indicate that an accelerator or brakes are used, that a steering wheel is turned and/or that a driver assistance system has been disabled, indicating that there will be substantial impact force, exceeding a predetermined value.

Based on the predicted 12 and/or determined 13 adverse conditions, three options are presented to schedule and/or perform medical scans. According to a first option 14, the scheduling and/or performing of the medical scans is done such that the medical scans that are most sensitive to adverse conditions are performed when the least adverse conditions are predicted and/or determined. For this, the sensitivity of the medical scans on the adverse conditions has been determined beforehand. In particular, there may be scans with different resolutions that have different sensitivity on the adverse conditions and/or even the scans from one scan sequence may have different sensitivity on the adverse conditions. As an example, when it is determined and/or predicted that the medical vehicle is or will be stopped, e.g., by information provided that a parking brake is engaged or by information provided by the navigation system that a traffic jam is ahead, the medical scan having the most sensitivity to impact forces will be performed at and/or scheduled for that time. As another example, when moderate impact forces are determined and/or predicted, a scan having lesser sensitivity to impact forces will be performed at and/or scheduled for that time. And when substantial impact forces are determined and/or predicted, the medical scans may be suspended for the duration of said substantial impact forces. Hence, an optimal match between the medical scans and the adverse conditions is found and an optimal quality of the medical scans is achieved.

According to a second option 15, the scheduling and/or performing of the medical scans is done such that that medical scan out of remaining medical scans, in particular of a scan sequence, is performed that has, among those medical scans that are allowed by the predicted and/or determined adverse conditions, the highest sensitivity to adverse conditions. In other words, based on the determined and/or predicted adverse conditions, it is determined which medical scans of the remaining scans are allowed to be performed, and among those medical scans, the one is chosen that has the highest sensitivity to the adverse conditions. In this context, "allowed" to be performed may mean that the medical scan may be performed without loss of quality compared to the same medical scan at ideal conditions, i.e., without adverse conditions. Hence, all of the medical scans that have been performed have the highest possible quality, but some of the medical scans of the scan sequence may not have been performed at all. These scans may, however, then be performed at, for example, a hospital, such that the scan sequence is completed.

According to a third option 16, the scheduling and/or performing of the medical scans is done such that the adverse conditions are most comparable to adverse conditions of a previous scan. In particular, the new medical scan is then an update scan or a comparison scan, to assess possible changes that may have occurred since the previous scan. When the most comparable adverse conditions are chosen for the update scan, the impact of the adverse conditions on the medical scans are similar such that the update scan can most easily be compared to the previous scan.

According to another option for operating the medical vehicle 1, settings of the medical vehicle 1 are adapted 17 in dependence on the obtained sensor readings. As an example, vibration compensators may be activated when certain vibrations are detected or when the r.p.m. of the engine are in a range known to cause resonant vibrations. As another example, the vehicle speed may be limited such that road bumps or curves lead to smaller impact forces. As yet another example, a vehicle route may be changed such that the vehicle travels on a smoother and/or less curvy road. As yet another example, settings of the vehicle suspension system 7 may be adjusted in order to lessen the impact forces caused by road bumps. As yet another example, settings of the anchoring system 8 that anchors the medical imaging system 4 to the compartment 3, may be adjusted. Said anchoring system 8 may be, for example, in a locking mode where the medical imaging system 4 is locked to the compartment 3, in a suspension mode where there is a suspension such as a spring suspension that allows limited motion of the medical imaging system 4 and absorbs some of the impact forces that act on the compartment 3, or in a damping mode, where said suspension is complemented by a damping system such as shock absorbers to prevent oscillations. If both the vehicle suspension system 7 and the anchoring system 8 are adjusted, a control unit may be used to coordinate the adjustments of these systems, in particular in order to avoid overcompensation and to avoid resonant oscillations of the medical imaging system 4. As yet another example, environmental conditioning settings, e.g., air conditioning settings, of the vehicle may be adjusted when temperatures and/or humidities have been determined to be out of a predetermined range.

According to yet another option for operating the medical vehicle, a movement of the compartment 3, the medical imaging system 4, a part of the medical imaging system and/or the patient is determined 18 based on the obtained sensor readings. In particular, said determination of a movement may be performed by an accelerometer and/or an inertial measurement unit 5.2. The determined acceleration can then be used to compute the movement of the compartment 3 and based on the movement of the compartment 3, a movement of the medical imaging system 4, of parts of the medical imaging system and/or of the patient may be inferred. A movement of the patient with respect to the medical imaging system 4 would naturally affect the medical scans, but also a movement of parts of the medical imaging system may lead to deformations of the medical imaging system 4 and hence, e.g., to a shift of a focus point.

The determined movement of the compartment, the medical imaging system, the part of the medical imaging system and/or the patient may then be used to dynamically adapt 19 a medical image formation. As an example, said dynamical adaptation may comprise a compensation calculation based on the determined movement. As another example, said dynamical adaptation may comprise an active shift of the patient to counteract the determined movement.

Alternatively, or additionally, the determined movement of the compartment 3, the medical imaging system 4, the part of the medical imaging system and/or the patient may be stored 20 for use for compensation of the movement in post processing of the medical images. Hence, the determined movements can be retrospectively be compensated for.

A selection of which ones of the steps of the method 9 are applied in a case at hand may be made manually, i.e., by an operator of the medical vehicle 1 or of the medical imaging system 4, using a predefined priority sequence and/or using an algorithm, in particular an algorithm that is optimized by artificial intelligence. Said selection methods may further depend on the patient, the medical condition of the patient and/or details of the medical imaging to be performed on the patient.

While the invention has been illustrated and described in detail in the drawing and foregoing description, such illustration and description are to be considered illustrative or exemplary and not restrictive; the invention is not limited to the disclosed embodiments. In particular, several embodiments may be combined to provide optimal limitation of gyroscopic forces.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. Any reference signs in the claims should not be construed as limiting the scope.

### LIST OF REFERENCE SIGNS:

- 1: medical vehicle
- 2: base vehicle
- 3: compartment
- 4: medical imaging system
- 5: base vehicle sensors
- 5.1: camera
- 5.2: inertial measurement unit
- 5.3: temperature sensor
- 6: vehicle management system
- 7: active suspension
- 8: anchoring system
- 9: method
- 10: obtaining sensor readings
- 11: performing medical scans
- 12: predicting adverse conditions
- 13: determining adverse conditions
- 14: first option for scheduling medical scans
- 15: second option for scheduling medical scans
- 16: third option for scheduling medical scans
- 17: adapting settings of the medical vehicle
- 18: determining a movement
- 19: dynamically adapting a medical image formation
- 20: storing the determined movement

## Claims

1. Medical vehicle (1) comprising a base vehicle (2) and a compartment (3) with a medical imaging system (4), wherein the compartment (3) is a part of the base vehicle (2) or the compartment (3) is attached to the base vehicle (2), wherein the base vehicle (2) comprises at least one base vehicle sensor (5) and wherein the medical vehicle (1) is configured to
obtain sensor readings from the at least one base vehicle sensor (5) during operation of the medical vehicle (1); and
perform medical scans of a patient during operation of the medical vehicle (1) in dependence on the obtained sensor readings.

2. Medical vehicle (1) according to claim 1, wherein the base vehicle (2) is a truck, a train, a plane, a helicopter, an autonomous flight object and/or a ship.

3. Medical vehicle (1) according to claim 1 or 2, wherein the medical imaging system (4) is a magnetic resonance imaging, MRI, a computed tomography, CT, a digital X-ray radiogrammetry, DXR, a single-photon emission computed tomography, SPECT, a positron emission tomography, PET, a dark field X-ray imaging, a small dedicated medical imaging and/or an ultrasound system.

4. Medical vehicle (1) according to any of claims 1 to 3, wherein the at least one base vehicle sensor (5) is at least one out of a group, the group consisting of a vehicle navigation system, an autonomous driving system, a board computer, a compass, a camera (5.1), a radar, a lidar, a speedometer, a wind sensor, a wave sensor, an acceleration sensor, an inertial measurement unit (5.2), a motion control sensor, an engine control sensor, a vibration sensor, a temperature sensor (5.3), a humidity sensor and/or an electromagnetic field sensor.

5. Medical vehicle (1) according to any of claims 1 to 4, wherein performing medical scans during operation of the medical vehicle (1) in dependence on the sensor readings comprises:
predicting, based on the obtained sensor readings, adverse conditions on the compartment (3); and
scheduling, based on the predicted adverse conditions, medical scans.

6. Medical vehicle (1) according to any of claims 1 to 5, wherein performing medical scans during operation of the medical vehicle (1) in dependence on the sensor readings comprises:
determining, based on the obtained sensor readings, current adverse conditions on the compartment (3); and
performing and/or dynamically adapting medical scans based on the determined current adverse conditions.

7. Medical vehicle (1) according to claim 5 or 6, wherein the scheduling and/or performing of the medical scans is done such that the medical scans that are most sensitive to adverse conditions are performed when the least adverse conditions are predicted and/or determined.

8. Medical vehicle (1) according to claim 5 or 6, wherein the scheduling and/or performing of the medical scans is done such that that medical scan out of remaining medical scans, in particular of a scan sequence, is performed that has, among those medical scans that are allowed by the predicted and/or determined adverse conditions, the highest sensitivity to adverse conditions.

9. Medical vehicle (1) according to claim 5 or 6, wherein the scheduling and/or performing of the medical scans is done such that the adverse conditions are most comparable to adverse conditions of a previous scan.

10. Medical vehicle (1) according to any of claims 1 to 9, wherein performing medical scans during operation of the medical vehicle (1) in dependence on the obtained sensor readings comprises adapting settings of the medical vehicle (1) in dependence on the obtained sensor readings.

11. Medical vehicle (1) according to any of claims 1 to 10, wherein the medical vehicle (1) is further configured to prevent the change of vehicle settings while an extra sensitive scan is performed.

12. Medical vehicle (1) according to any of claims 1 to 11, wherein performing medical scans during operation of the medical vehicle (1) in dependence on the obtained sensor readings comprises determining a movement of the compartment (3), the medical imaging system (4), a part of the medical imaging system (4) and/or the patient based on the obtained sensor readings.

13. Medical vehicle (1) according to claim 12, wherein the medical vehicle (1) is further configured to dynamically adapt a medical image formation based on the determined movement of the compartment (3), the medical imaging system (4), the part of the medical imaging system (4) and/or the patient.

14. Medical vehicle (1) according to claim 12 or 13, wherein the medical vehicle (1) is further configured to store the determined movement of the compartment (3), the medical imaging system (4), the part of the medical imaging system (4) and/or the patient for use for compensation of the movement in post processing of the medical images.

15. Method for operating a medical vehicle (1) according to any of claims 1 to 14, comprising:
obtaining sensor readings from the at least one base vehicle sensor (5) during operation of the medical vehicle (1); and
performing medical scans of the patient during operation of the medical vehicle (1) in dependence on the obtained sensor readings.
